# EUROPEAN PATENT APPLICATION

(11) **EP 4 311 536 A1**
(43) Date of publication of application: **31.01.2024**
(21) Application number: 23187553.5
(22) Date of filing: 25.07.2023
(51) Int. Cl.: A61K 6/90, C08L 5/04, B29C 33/60

(54) **DENTAL SEPARATION MATERIAL**

(30) Priority: 29.07.2022 JP 2022121926
(71) Applicant: GC Corporation, Sunto-gun, Shizuoka 410-1307 (JP)
(72) Inventor: SHIBUYA, Yuki, Tokyo, 174-8585 (JP); FUJIMI, Atsushi, Tokyo, 174-8585 (JP); NIIZEKI, Naofumi, Tokyo, 174-8585 (JP)
(74) Representative: Müller-Boré & Partner Patentanwälte PartG mbB

(57) **Abstract**

A dental separation material which prevents adhesion of an impression material to a tooth is provided. The dental separation material includes a fatty acid and an aqueous solvent.

## Description

### TECHNICAL FIELD

The present invention relates to a dental separation material.

### BACKGROUND ART

Conventionally, separation materials used in dentistry are generally dental separation materials for resin for the purpose of separating a gypsum model from a cured polymerizable resin. For example, in dentistry, there is known a resin-use dental separation material that prevents adhesion of a temporary restoration by polymerizing and curing a polymerizable resin on a cured film of a bonding material (for example, Patent Document 1).

### [Related-Art Documents]

### [Patent Documents]

[Patent Document 1] Japanese Patent No. 2783627

### SUMMARY OF THE INVENTION

### [Problems to be Solved by the Invention]

A dental separation material that prevents an impression material from adhering to teeth has been desired.

An object of the present invention is to provide a dental separation material that prevents an impression material from adhering to teeth and enables highly accurate impression taking.

### [Means for solving the problem]

One aspect of the present invention is a dental separation material including a fatty acid and an aqueous solvent.

### [Effects of the Invention]

According to one aspect of the present invention, a dental separation material that prevents an impression material from adhering to teeth and enables highly accurate impression taking can be provided.

### DETAILED DESCRIPTION OF THE INVENTION

Hereinafter, embodiments for carrying out the present invention will be described.

### <Dental Separation Material>

The dental separation material of the present embodiment contains a fatty acid and an aqueous solvent. In the present specification, the dental separation material refers to a material that is applied to or sprayed onto teeth before a dental material is attached to the teeth in order to prevent a part of the dental material from sticking to or adhering to the teeth when the dental material attached to the teeth (or tooth) is removed for dental use.

### [Fatty Acid]

The fatty acid is a carboxylic acid having a carboxyl group at one end where carbon atoms are linked in a chain. The fatty acid is not particularly limited, but is preferably a carbon number of a fatty acid being 6 or more, and more preferably a carbon number of a fatty acid being 10 or more (higher fatty acid).

The kind of the fatty acid is not particularly limited, and may be either a saturated fatty acid or an unsaturated fatty acid, but is preferably a saturated fatty acid.

Examples of the saturated fatty acid include octylic acid, octanoic acid, lauric acid, myristic acid, palmitic acid, stearic acid, docosanoic acid (behenic acid), tetracosanoic acid (lignoceric acid), and the like. Among them, lauric acid, myristic acid, palmitic acid, stearic acid, and docosanoic acid (behenic acid) are preferably used. These saturated fatty acids may be used alone or in combination of two or more saturated fatty acids.

Examples of unsaturated fatty acids include oleic acid, linoleic acid, α-linolenic acid, γ-linolenic acid, arachidonic acid, icosapentaenoic acid, docosahexaenoic acid, and the like. Among them, oleic acid and linoleic acid are preferably used. These unsaturated fatty acids may be used alone or in combination of two or more unsaturated fatty acids.

As the fatty acid, one or more saturated fatty acids and one or more unsaturated fatty acids may be used in combination.

A content of the fatty acid in the dental separation material is not particularly limited, but is in a range from 0.01% by mass to 65% by mass inclusive, preferably in a range from 0.05% by mass to 60% by mass inclusive, and more preferably in a range from 0.1% by mass to 55% by mass inclusive.

### [Aqueous Solvent]

The aqueous solvent refers to water, a water-soluble solvent or a mixture thereof. As the water, ion-exchanged water or tap water may be used.

Examples of the water-soluble solvents include monoalcohols such as methanol, ethanol, 1-propanol, 2-propanol, 1-butanol, 2-butanol, and the like; polyols such as ethylene glycol, 1,2-propylene glycol, 1,3-propylene glycol, 1,2-butylene glycol, 1,3-butylene glycol, 1,4-butylene glycol, 2,3-butylene glycol, 1,2-pentanediol, 1,4-pentanediol, 1,5-pentanediol, 2,4-pentanediol, glycerin, polyethylene glycol, dipropylene glycol, polypropylene glycol, and the like; polyol ethers such as ethylene glycol monoethyl ether, ethylene glycol monopropyl ether, ethylene glycol monobutyl ether, ethylene glycol dimethyl ether, and the like; and the like. These water-soluble solvents may be used alone or in combination of two or more water-soluble solvents.

A content of the aqueous solvent is not particularly limited, but is 35% by mass or more, preferably 40% by mass or more, and more preferably 45% by mass or more.

The dental separation material may contain other components (for example, a surfactant and the like).

Since the dental separation material of the present embodiment contains the fatty acid and the aqueous solvent as described above, it is possible to prevent a part of the dental material from adhering to or remaining on the tooth when the dental material attached to the teeth is removed.

In addition, in the dental separation material of the present embodiment, adhesion of the dental material to the tooth can be prevented, and as a result, by taking an impression of the teeth using such a dental separation material, highly accurate impression taking can be performed.

Since the dental separation material of the present embodiment contains a carbon number of the fatty acid being 6 or more as described above, it is possible to enhance the effect of preventing a part of the dental material from adhering to or remaining on the tooth when the dental material attached to the teeth is removed, and it is possible to perform impression taking with higher accuracy.

In the dental separation material of the present embodiment, the saturated fatty acid is contained as the fatty acid as described above, so that the dental separation material can be easily removed when the dental separation material is removed from the tooth.

### <Dental Impression Material>

The dental separation material of the present embodiment is used for dental impression materials. In the present specification, the dental impression material refers to a material that molds the surface of an object in the oral cavity (also referred to as taking an impression). The form of the dental impression material is not particularly limited, and examples thereof include an alginate impression material, an agar impression material, a silicone impression material, and the like. However, this dental separation material is preferably used with alginate impression material.

When the dental separation material of the present embodiment is used for a dental impression material, it is possible to prevent a part of the impression material from adhering to or remaining on a tooth when the impression material attached to the teeth is removed. In addition, since the adhesion of the impression material to the teeth can be prevented, highly accurate impression taking can be performed.

In addition, when the dental impression material to which the dental separation material of the present embodiment is applied is an alginate impression material, the effect of preventing the adhesion of the impression material and the effect of obtaining an impression with high accuracy are remarkably obtained.

### [Examples]

Hereinafter, the present invention will be described more specifically with reference to Examples. Examples and Comparative Examples were evaluated by the following tests. In the following description, numerical values without a unit are based on mass (parts by mass) unless otherwise specified.

### <Separation Effect Test of Dental Alginate Impression Material>

A cross-section of a bovine tooth embedded in a dental cold-polymerization resin (UNIFAST (Registered Trademark) II, manufactured by GC Corporation) was polished with #600 water-resistant polishing paper. The separation material was applied to the polished surface of the bovine tooth using an applicator and dried with air. 1.5 mL of a dental alginate impression material (AROMA INJECTION (Registered Trademark), manufactured by GC Corporation) was extruded onto the dried surface of the tooth and pressed with an acrylic plate, resulting in the forming of a sample. The sample was left in a constant-temperature water bath at 35°C for a length of time equal to the length of time being retained in the oral cavity, for example, 1.5 minutes. The sample was taken out of the constant-temperature water bath, peeled off from the bovine tooth together with the acrylic plate, and the surface of tooth was observed.

The separation effect was evaluated as "good" when no dental alginate impression material remained on the tooth surface, and as "poor" when the dental alginate impression material remained on the tooth surface. A case where the separation effect test could not be performed because the separation material became white particles and could not be applied to the tooth was evaluated as "impracticable".

### <Impression Accuracy>

Impression accuracy was evaluated by the following procedure using a test block for a detail reproduction test in accordance with JIS T 6505:2016, 7.3. First, the separation material was applied to the test block using an applicator and dried with air. 1.5 mL of a dental alginate impression material was extruded onto the dried surface of the test block and pressed with an acrylic plate, resulting in the forming of a sample. The sample was left in a constant-temperature water bath at 35°C for a length of time equal to the length of time being retained in the oral cavity. The sample was taken out of the constant-temperature water bath, peeled off from the test block together with the acrylic plate, and the surface of test block was observed.

The impression accuracy was evaluated as "good" when a thin line of 20 um was confirmed on the surface of the test block, and as "poor" when a fine line was not confirmed on the surface of the test block. A case where the impression accuracy test could not be performed because the dental alginate impression material remained on the surface (could not be separated) in the above separation effect test was evaluated as "impracticable".

### <Removability of Separation Material>

After the test of the separation effect of the dental alginate impression material, it was evaluated whether or not a film of the separation material on the tooth surface could be removed by the following procedure. First, the surface of the bovine tooth after the test was washed by spraying water using a three-way syringe. In the same manner as in the above-described test of the separation effect, 1.5 mL of a dental alginate impression material was extruded onto the surface of bovine tooth and pressed with an acrylic plate, resulting in the forming of a sample. The sample was left in a constant-temperature water bath at 35°C for a length of time equal to the length of time being retained in the oral cavity. The sample was taken out of the constant-temperature water bath, peeled off from the bovine tooth surface together with the acrylic plate, and the surface of bovine tooth was observed.

The removability was evaluated as "good" when the dental alginate impression material remained on the surface of the bovine tooth, and as "poor" when the dental alginate impression material did not remain on the surface of the bovine tooth. In the above-described separation effect test, a case where the removability test could not be performed because the dental alginate impression material remained on the surface of the bovine tooth (could not be separated) in the above separation effect test was evaluated as "impracticable".

Examples and Comparative Examples are indicated below.

### [Example 1]

A separation material containing 50 parts by mass of lauric acid as a fatty acid and 50 parts by mass of ethanol as an aqueous solvent was prepared and evaluated. The formulation and evaluation results of Example 1 are indicated in Table 1.

### [Example 2]

A separation material was prepared and evaluated in the same manner as in Example 1, except that the amount of lauric acid was adjusted to 40 parts by mass and the amount of ethanol was adjusted to 60 parts by mass. The formulation and evaluation results of Example 2 are indicated in Table 1.

### [Example 3]

A separation material was prepared and evaluated in the same manner as in Example 1, except that the amount of lauric acid was adjusted to 30 parts by mass and the amount of ethanol was adjusted to 70 parts by mass. The formulation and evaluation results of Example 3 are indicated in Table 1.

### [Example 4]

A separation material was prepared and evaluated in the same manner as in Example 1, except that the amount of lauric acid was adjusted to 20 parts by mass and the amount of ethanol was adjusted to 80 parts by mass. The formulation and evaluation results of Example 4 are indicated in Table 1.

### [Example 5]

A separation material was prepared and evaluated in the same manner as in Example 1, except that the amount of lauric acid was adjusted to 10 parts by mass and the amount of ethanol was adjusted to 90 parts by mass. The formulation and evaluation results of Example 5 are indicated in Table 1.

### [Example 6]

A separation material was prepared and evaluated in the same manner as in Example 1, except that the amount of lauric acid was adjusted to 5 parts by mass and the amount of ethanol was adjusted to 95 parts by mass. The formulation and evaluation results of Example 6 are indicated in Table 1.

### [Example 7]

A separation material was prepared and evaluated in the same manner as in Example 1, except that the amount of lauric acid was adjusted to 2 parts by mass and the amount of ethanol was adjusted to 98 parts by mass. The formulation and evaluation results of Example 7 are indicated in Table 1.

### [Example 8]

A separation material was prepared and evaluated in the same manner as in Example 1, except that the amount of lauric acid was adjusted to 1 part by mass and the amount of ethanol was adjusted to 99 parts by mass. The formulation and evaluation results of Example 8 are indicated in Table 1.

### [Example 9]

A separation material was prepared and evaluated in the same manner as in Example 3, except that 30 parts by mass of myristic acid was used instead of lauric acid. The formulation and evaluation results of Example 9 are indicated in Table 1.

### [Example 10]

A separation material was prepared and evaluated in the same manner as in Example 9, except that the amount of myristic acid was adjusted to 20 parts by mass and the amount of ethanol was adjusted to 80 parts by mass. The formulation and evaluation results of Example 10 are indicated in Table 1.

### [Example 11]

A separation material was prepared and evaluated in the same manner as in Example 9, except that the amount of myristic acid was adjusted to 10 parts by mass and the amount of ethanol was adjusted to 90 parts by mass. The formulation and evaluation results of Example 11 are indicated in Table 2.

### [Example 12]

A separation material was prepared and evaluated in the same manner as in Example 9, except that the amount of myristic acid was adjusted to 5 parts by mass and the amount of ethanol was adjusted to 95 parts by mass. The formulation and evaluation results of Example 12 are indicated in Table 2.

### [Example 13]

A separation material was prepared and evaluated in the same manner as in Example 9, except that the amount of myristic acid was adjusted to 2 parts by mass and the amount of ethanol was adjusted to 98 parts by mass. The formulation and evaluation results of Example 13 are indicated in Table 2.

### [Example 14]

A separation material was prepared and evaluated in the same manner as in Example 9, except that the amount of myristic acid was adjusted to 1 part by mass and the amount of ethanol was adjusted to 99 parts by mass. The formulation and evaluation results of Example 14 are indicated in Table 2.

### [Example 15]

A separation material was prepared and evaluated in the same manner as in Example 9, except that the amount of myristic acid was adjusted to 0.5 parts by mass and the amount of ethanol was adjusted to 99.5 parts by mass. The formulation and evaluation results of Example 15 are indicated in Table 2.

### [Example 16]

A separation material was prepared and evaluated in the same manner as in Example 5, except that 10 parts by mass of palmitic acid was used instead of lauric acid. The formulation and evaluation results of Example 16 are indicated in Table 2.

### [Example 17]

A separation material was prepared and evaluated in the same manner as in Example 16, except that the amount of palmitic acid was adjusted to 5 parts by mass and the amount of ethanol was adjusted to 95 parts by mass. The formulation and evaluation results of Example 17 are indicated in Table 2.

### [Example 18]

A separation material was prepared and evaluated in the same manner as in Example 16, except that the amount of palmitic acid was adjusted to 2 parts by mass and the amount of ethanol was adjusted to 98 parts by mass. The formulation and evaluation results of Example 18 are indicated in Table 2.

### [Example 19]

A separation material was prepared and evaluated in the same manner as in Example 16, except that the amount of palmitic acid was adjusted to 1 part by mass and the amount of ethanol was adjusted to 99 parts by mass. The formulation and evaluation results of Example 19 are indicated in Table 2.

### [Example 20]

A separation material was prepared and evaluated in the same manner as in Example 16, except that the amount of palmitic acid was adjusted to 0.5 parts by mass and the amount of ethanol was adjusted to 99.5 parts by mass. The formulation and evaluation results of Example 20 are indicated in Table 2.

### [Example 21]

A separation material was prepared and evaluated in the same manner as in Example 7, except that 2 parts by mass of stearic acid was used instead of lauric acid. The formulation and evaluation results of Example 21 are indicated in Table 3.

### [Example 22]

A separation material was prepared and evaluated in the same manner as in Example 21, except that the amount of stearic acid was adjusted to 1 part by mass and the amount of ethanol was adjusted to 99 parts by mass. The formulation and evaluation results of Example 22 are indicated in Table 3.

### [Example 23]

A separation material was prepared and evaluated in the same manner as in Example 21, except that the amount of stearic acid was adjusted to 0.5 parts by mass and the amount of ethanol was adjusted to 99.5 parts by mass. The formulation and evaluation results of Example 23 are indicated in Table 3.

### [Example 24]

A separation material was prepared and evaluated in the same manner as in Example 23, except that 0.5 parts by mass of docosanoic acid was used instead of stearic acid. The formulation and evaluation results of Example 24 are indicated in Table 3.

### [Example 25]

A separation material was prepared and evaluated in the same manner as in Example 24, except that the amount of docosanoic acid was adjusted to 0.1 parts by mass and the amount of ethanol was adjusted to 99.9 parts by mass. The formulation and evaluation results of Example 25 are indicated in Table 3.

### [Example 26]

A separation material was prepared and evaluated in the same manner as in Example 8, except that 1 part by mass of oleic acid (unsaturated fatty acid) was used instead of lauric acid. The formulation and evaluation results of Example 26 are indicated in Table 3.

### [Example 27]

A separation material was prepared and evaluated in the same manner as in Example 8, except that 1 part by mass of linoleic acid (unsaturated fatty acid) was used instead of lauric acid. The formulation and evaluation results of Example 27 are indicated in Table 3.

### [Comparative Example 1]

A separation material was prepared and evaluated in the same manner as in Example 8, except that 1 part by mass of isopropyl myristate (fatty acid ester) was used instead of lauric acid. The formulation and evaluation results of Comparative Example 1 are indicated in Table 3.

### [Comparative Example 2]

A separation material was prepared and evaluated in the same manner as in Example 8, except that 1 part by mass of allyl hexanoate (short chain fatty acid ester) was used instead of lauric acid. The formulation and evaluation results of Comparative Example 2 are indicated in Table 3.

### [Comparative Example 3]

A separation material was prepared and evaluated in the same manner as in Example 1, except that 100 parts by mass of lauric acid was used (no aqueous solvent was added). The formulation and evaluation results of Comparative Example 3 are indicated in Table 3.

**[Table 1]**

| Type | Materials | Examples | | | | | | | | | |
|---|---|---|---|---|---|---|---|---|---|---|---|
| | | 1 | 2 | 3 | 4 | 5 | 6 | 7 | 8 | 9 | 10 |
| Solvents | ethanol | 50 | 60 | 70 | 80 | 90 | 95 | 98 | 99 | 70 | 80 |
| Saturated fatty acid (C12:0) | lauric acid | 50 | 40 | 30 | 20 | 10 | 5 | 2 | 1 | | |
| Saturated fatty acid (C14:0) | myristic acid | | | | | | | | | 30 | 20 |
| Saturated fatty acid (C16:0) | palmitic acid | | | | | | | | | | |
| Saturated fatty acid (C18:0) | stearic acid | | | | | | | | | | |
| Saturated fatty acid (C22:0) | docosanoic acid | | | | | | | | | | |
| Unsaturated fatty acid (C18:1) | oleic acid | | | | | | | | | | |
| Unsaturated fatty acid (C18:2) | linoleic acid | | | | | | | | | | |
| Fatty acid ester | isopropyl myristate | | | | | | | | | | |
| Fatty acid ester | allyl hexanoate | | | | | | | | | | |
| Total | | 100 | 100 | 100 | 100 | 100 | 100 | 100 | 100 | 100 | 100 |
| Evaluations | Separation effect | Good | Good | Good | Good | Good | Good | Good | Good | Good | Good |
| | Impression accuracy | Good | Good | Good | Good | Good | Good | Good | Good | Good | Good |
| | Removability of separation material | Good | Good | Good | Good | Good | Good | Good | Good | Good | Good |

**[Table 2]**

| Type | Materials | Examples | | | | | | | | | |
|---|---|---|---|---|---|---|---|---|---|---|---|
| | | 11 | 12 | 13 | 14 | 15 | 16 | 17 | 18 | 19 | 20 |
| Solvents | ethanol | 90 | 95 | 98 | 99 | 99.5 | 90 | 95 | 98 | 99 | 99.5 |
| Saturated fatty acid (C12:0) | lauric acid | | | | | | | | | | |
| Saturated fatty acid (C14:0) | myristic acid | 10 | 5 | 2 | 1 | 0.5 | | | | | |
| Saturated fatty acid (C16:0) | palmitic acid | | | | | | 10 | 5 | 2 | 1 | 0.5 |
| Saturated fatty acid (C18:0) | stearic acid | | | | | | | | | | |
| Saturated fatty acid (C22:0) | docosanoic acid | | | | | | | | | | |
| Unsaturated fatty acid (C18:1) | oleic acid | | | | | | | | | | |
| Unsaturated fatty acid (C18:2) | linoleic acid | | | | | | | | | | |
| Fatty acid ester | isopropyl myristate | | | | | | | | | | |
| Fatty acid ester | allyl hexanoate | | | | | | | | | | |
| Total | | 100 | 100 | 100 | 100 | 100 | 100 | 100 | 100 | 100 | 100 |
| Evaluations | Separation effect | Good | Good | Good | Good | Good | Good | Good | Good | Good | Good |
| | Impression accuracy | Good | Good | Good | Good | Good | Good | Good | Good | Good | Good |
| | Removability of separation material | Good | Good | Good | Good | Good | Good | Good | Good | Good | Good |

**[Table 3]**

| Type | Materials | Examples | | | | | | | Comparative Examples | | |
|---|---|---|---|---|---|---|---|---|---|---|---|
| | | 21 | 22 | 23 | 24 | 25 | 26 | 27 | 1 | 2 | 3 |
| Solvents | ethanol | 98 | 99 | 99.5 | 99.5 | 99.9 | 99 | 99 | 99 | 99 | |
| Saturated fatty acid (C12:0) | lauric acid | | | | | | | | | | 100 |
| Saturated fatty acid (C14:0) | myristic acid | | | | | | | | | | |
| Saturated fatty acid (C16:0) | palmitic acid | | | | | | | | | | |
| Saturated fatty acid (C18:0) | stearic acid | 2 | 1 | 0.5 | | | | | | | |
| Saturated fatty acid (C22:0) | docosanoic acid | | | | 0.5 | 0.1 | | | | | |
| Unsaturated fatty acid (C18:1) | oleic acid | | | | | | 1 | | | | |
| Unsaturated fatty acid (C18:2) | linoleic acid | | | | | | | 1 | | | |
| Fatty acid ester | isopropyl myristate | | | | | | | | 1 | | |
| Fatty acid ester | allyl hexanoate | | | | | | | | | 1 | |
| Total | | 100 | 100 | 100 | 100 | 100 | 100 | 100 | 100 | 100 | 100 |
| Evaluations | Separation effect | Good | Good | Good | Good | Good | Good | Good | Poor | Poor | Impracticable |
| | Impression accuracy | Good | Good | Good | Good | Good | Good | Good | Impracticable | Impracticable | Impracticable |
| | Removability of separation material | Good | Good | Good | Good | Good | Poor | Poor | Impracticable | Impracticable | Impracticable |

From Tables 1 to 3, the separation materials in which the fatty acid and the aqueous solvent were used were "good" in both the separation effect and the impression accuracy (Examples 1 to 27). In addition, the separation materials in which the saturated fatty acid was used exerted good removability (Examples 1 to 25).

On the other hand, in the case where the fatty acid was not used and the case where the aqueous solvent was not used, the separation effect was poor or the test of the separation effect was not performable (Comparative Examples 1 to 3).

The embodiments disclosed above include, for example, the following aspects.
<1> A dental separation material includes a fatty acid and an aqueous solvent.
<2> The dental separation material according to <1>, wherein a carbon number of the fatty acid is 6 or more.
<3> The dental separation material according to <1> or <2>, wherein the fatty acid is a saturated fatty acid.
<4> The dental separation material according to any one of <1> to <3>, wherein the dental separation material is configured to be used for a dental impression material.
<5> The dental separation material according to <4>, wherein the dental impression material is an alginate impression material.

Although the embodiments of the present invention have been described above, the present invention is not limited to the specific embodiments, and various modifications and changes can be made within the scope of the invention described in the claims.

This patent application is based on and claims priority to Japanese Patent Application No. 2022-121926 filed on July 29, 2022, the entire contents of which are incorporated herein by reference.

## Claims

1. A dental separation material comprising:
a fatty acid; and
an aqueous solvent.

2. The dental separation material according to claim 1, wherein a carbon number of the fatty acid is 6 or more.

3. The dental separation material according to claim 1, wherein the fatty acid is a saturated fatty acid.

4. The dental separation material according to any one of claims 1 to 3, wherein the dental separation material is configured to be used for a dental impression material.

5. The dental separation material according to claim 4, wherein the dental impression material is an alginate impression material.
